# EUROPÄISCHE PATENTANMELDUNG

(11) **EP 2 437 060 A1**
(43) Veröffentlichungstag der Anmeldung: **04.04.2012**
(21) Anmeldenummer: 10186327.2
(22) Anmeldetag: 01.10.2010
(51) Int. Cl.: G01N 33/564, G01N 33/68, C07K 17/00

(54) **Markersequenzen für Multiple Sklerose und deren Verwendung**

(71) Anmelder: Protagen AG, 44227 Dortmund (DE)
(72) Erfinder: Lüking, Angelika, 44892 Bochum (DE); Kowald, Axel, 44892 Bochum (DE); Göhler, Heike, 44797 Bochum (DE)
(74) Vertreter: Simandi, Claus

(57) **Zusammenfassung**

Die vorliegende Erfindung betrifft neue Markersequenzen für Multiple Sklerose und deren diagnostische Verwendung samt einem Verfahren zum Screenen von potentiellen Wirkstoffen für Multiple Sklerose - Erkrankungen mittels dieser Markersequenzen. Ferner betrifft die Erfindung eine diagnostische Vorrichtung enthaltend solche Markersequenzen für Multiple Sklerose, insbesondere ein Proteinbiochip und dessen Verwendung.

## Beschreibung

Die vorliegende Erfindung betrifft neue Markersequenzen für Multiple Sklerose und deren diagnostische Verwendung samt einem Verfahren zum Screenen von potentiellen Wirkstoffen für Multiple Sklerose - Erkrankungen mittels dieser Markersequenzen. Ferner betrifft die Erfindung eine diagnostische Vorrichtung enthaltend solche Markersequenzen für Multiple Sklerose, insbesondere ein Proteinbiochip und dessen Verwendung.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Es besteht jedoch ein hohes Bedürfnis indikationsspezifische diagnostische Vorrichtungen, wie einen Proteinbiochip, bereitzustellen.

Die Aufgabe der vorliegenden Erfindung ist die Bereitstellung von verbesserten Markersequenzen und deren diagnostische Verwendung zur Behandlung von Multiple Sklerose.

Die Bereitstellung von spezifischen Markersequenzen erlaubt eine sichere Diagnose und Stratifizierung von Patienten mit Multiple Sklerose, insbesondere mittels eines Proteinbiochips.

Daher betrifft die Erfindung die Verwendung von Markersequenzen zur Diagnose von Multiple Sklerose, wobei mindestens eine Markersequenz einer cDNA ausgewählt aus der Gruppe SEQ 1 - 308 oder jeweils ein dafür kodierendes Protein oder jeweils einer Teilsequenz oder Fragment davon (nachstehend: erfindungsgemäße Markersequenzen) an oder von einem zu untersuchenden Patienten bestimmt wird.

Die erfindungsgemäßen Markersequenzen konnten mittels differentiellem Screenen von Proben und zwar gesunder Probanden mit Patientenproben mit Multiple Sklerose identifiziert werden.

Hierbei konnte erstmals mittels Proteinbiochips (siehe Beispiele) diese erfindungsgemäßen Markersequenzen identifiziert werden.

Im Stand der Technik konnten zwar bereits Markersequenzen für Multiple Sklerose mit Hilfe eines Proteinbiochip identifiziert werden, siehe W02009030225. Vorliegend wird jedoch erfindungsgemäß eine verbesserte bioinformatorische Auswertung angestrengt und die Proben werden besonders bevorzugt aus dem Liquor cerebrospinalis (CSF) entnommen. Ferner werden speziell ausgesuchte Proben verwendet, die der hohen Empfindlichkeit eines Proteinbiochips entgegenkommen.

Der Begriff "Multiple Sklerose ((MS), auch Encephalomyelitis disseminata)" betrifft eine autoimmun-entzündliche / demyelinisierende und degenerative Erkrankung des Erkrankung des Zentralnervensystems (Definition z.B. nach Pschyrembel, de Gruyter, 261. Auflage (2007), Berlin).

Erfindungswesentlich ist, dass die Proben nicht aus üblichen Blutbanken entnommen werden, sondern von MS-Patienten sorgfältig ausgewählt wurden, die z.B. HIV und HCV negativ sind und insbesondere auf Infektionskrankheiten getestet wurden. Das aufwändige Probeselektionsverfahren erlaubt z.B. eine hinreichende vorteilhafte Abgrenzung von Erkrankungen wie eine zur MS Symptom-ähnlichen Neuroborelliose. Weiterhin werden falsch-positive Ergebnisse ausgeschlossen, zum einen aufgrund der strengen bioinformatorischen Auswertung (siehe Beispiele) als auch durch den Vergleich der Ergebnisse auf einem erfindungsgemäßen Proteinbiochip mit z.B. Seren von Neuroborelliose-Patienten, die kein Multiple Sklerose aufweisen.

Weiterhin erfolgt im Unterschied zur WO2009030225 die Herstellung der Proteinbiochips mittels Normalisierung von mindestens 1.000, vorzugsweise 2.000 verschiedenen oder mehr Autoantigenen des Menschen, die nicht indikationsspezifisch für Multiple Sklerose sind. Solche Autoantigene können z.B. aus anderen Körperflüssigkeiten von Patienten anderer Krankheiten gewonnen werden (z.B. Pankreaskrebs, Rheumatoide Arthritis, Prostata etc.).

Daher betrifft die Erfindung auch solche erfindungsgemäße indikationsspezifische Proteinbiochips zur Diagnose von Multiple Sklerose, wobei in einem weiteren Verifizierungsschritt, die auf dem Proteinbiochip repräsentierte Proteine mit Autoantikörper aus Nicht-Multiple Sklerose Patienten normalisiert werden und auf diese Weise falsch-positive Proteine entfernt werden können. Verbleibende nicht-falsch-positive Proteine können auf einem Proteinbiochip neu zusammengestellt werden, so genanntes Rearraying. Dies erlaubt ebenfalls den Ausschluss von Autoantikörpern, die auf E. coli positiv sind. Dies ist eine weitere qualitative Verbesserung, da Autoantikörper ausgeschlossen werden können, die z.B. gegen E. coli Darmbakterien im Menschen gerichtet sind. Infolge dessen können vorteilhaft bei einem verbesserten Signal/Rausch Verhältnis neue Markersequenzen identifiziert werden.

In einer weiteren bevorzugten Ausführungsform werden daher mindestens 2 bis 5 oder 10, vorzugsweise 30 bis 50 Markersequenzen oder 50 bis 100 oder mehr Markersequenzen an oder von einem zu untersuchenden Patienten bestimmt.

In einer weiteren Ausführungsform der Erfindung können die erfindungsgemäßen Markersequenzen ebenfalls mit bekannten Biomarkern für diese Indikation kombiniert, ergänzt oder erweitert werden.

In einer bevorzugten Ausführungsform erfolgt die Bestimmung der Markersequenzen außerhalb des menschlichen Körpers und die Bestimmung erfolgt in einer ex vivo / in vitro Diagnose.

In einer weiteren Ausführungsform der Erfindung betrifft die Erfindung die Verwendung von Markersequenzen als Diagnostika, wobei mindestens eine Markersequenz einer cDNA ausgewählt aus der Gruppe SEQ 1 - 308 oder jeweils ein dafür kodierendes Protein oder jeweils einer Teilsequenz oder Fragment davon ist.

Ferner betrifft die Erfindung ein Verfahren zur Diagnose von Multiple Sklerose, wobei a.) mindestens eine Markersequenz einer cDNA ausgewählt aus der Gruppe SEQ 1 - 308 oder jeweils ein dafür kodierendes Protein oder jeweils einer Teilsequenz oder Fragment davon auf einem festen Träger aufgebracht wird und b.) mit Körperflüssigkeit oder Gewebeauszug eines Patienten in Kontakt gebracht wird und c.) der Nachweis einer Wechselwirkung der Körperflüssigkeit oder Gewebeauszug mit den Markersequenzen aus a.) erfolgt.

Daher betrifft die Erfindung ebenfalls Diagnostika zur Diagnose von Multiple Sklerose jeweils ausgewählt aus der Gruppe SEQ 1 - 308 oder jeweils ein dafür kodierendes Protein oder jeweils einer Teilsequenz oder Fragment davon.

Der Nachweis einer solchen Wechselwirkung kann beispielsweise durch eine Sonde, insbesondere durch einen Antikörper erfolgen.

Daher betrifft die Erfindung ebenfalls die Aufgabe eine diagnostische Vorrichtung oder einen Assay, insbesondere einen Proteinbiochip, bereitzustellen, der für die Multiple Sklerose eine Diagnose oder Untersuchung erlaubt.

Ferner betrifft die Erfindung ein Verfahren zum Stratifizieren, insbesondere zur Risikostratifizierung und / oder Therapiesteuerung eines Patienten mit Multiple Sklerose, wobei mindestens eine Markersequenz einer cDNA ausgewählt aus der Gruppe SEQ 1 - 308 oder jeweils ein dafür kodierendes Protein an einem zu untersuchenden Patienten bestimmt wird.

Ferner umfasst ist die Stratifizierung der Patienten mit Multiple Sklerose in neue oder etablierte Subgruppen der Multiple Sklerose, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

3Diagnose" im Sinne dieser Erfindung bedeutet die positive Feststellung der Multiple Sklerose mittels der erfindungsgemäßen Markersequenzen sowie die Zuordnung der Patienten zur Erkrankung an Multiple Sklerose. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose ebenfalls die Differentialdiagnose von Multiple Sklerose mittels der erfindungsgemäßen Markersequenzen sowie die Prognose der Multiple Sklerose.

"Stratifizieren (auch: Stratifikation) oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Multiple Sklerose untersucht wird.

Der Begriff "Markersequenzen" im Sinne dieser Erfindung bedeutet, dass die cDNA oder das jeweils daraus erhältliche Polypeptid oder Protein signifikant für Multiple Sklerose sind. Beispielsweise können die cDNA oder das jeweils daraus erhältliche Polypeptid oder Protein eine Wechselwirkung mit Substanzen aus der Körperflüssigkeit oder Gewebeauszug eines Patienten mit Multiple Sklerose aufweisen (z.B. Antigen (Epitop) / Antikörper (Paratop) Wechselwirkung). Im Sinne der Erfindung bedeutet "wobei mindestens eine Markersequenz einer cDNA ausgewählt aus der Gruppe SEQ 1 - 308 oder jeweils ein dafür kodierendes Protein oder jeweils einer Teilsequenz oder Fragment davon an einem zu untersuchenden Patienten bestimmt wird", dass eine Wechselwirkung zwischen der Körperflüssigkeit oder Gewebeauszuges eines Patienten und den erfindungsgemäßen Markersequenzen nachgewiesen wird. Eine solche Wechselwirkung ist z.B. eine Bindung, insbesondere eine bindende Substanz an mindestens einer erfindungsgemäßen Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

In einer weiteren Ausführungsform der Erfindung können jedoch die erfindungsgemäßen Markersequenzen in einer signifikant höheren oder niedrigeren Expressionsrate oder Konzentration vorliegen, dass auf die Multiple Sklerose hinweist. Hierbei wird mittels Proteomics oder Nukleinsäureblots die relativen Expressionsraten krank / gesund der erfindungsgemäßen Markersequenzen für Multiple Sklerose bestimmt.

Die Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen sind Gegenstand der Tabelle A und können durch den jeweilig zitierten Datenbankeintrag (auch mittels Internet:
http://www.ncbi.nlm.nih.gov/) eindeutig identifiziert werden (siehe in Tabelle A: dort Accession No.), siehe ebenfalls das zugehörige Sequenzprotokoll.

Daher betrifft die Erfindung ebenfalls die Volllängesequenzen der erfindungsgemäßen Marker und zwar wie in Tabelle A über den bekannten Datenbankeintrag definiert, nachstehend SEQ 1a-308a (cDNA) bzw. SEQ 1b-308b (Protein) genannt.

Weiterhin umfasst sind daher ebenfalls analoge Ausführungsformen von SEQ 1a-308a zu den Markersequenzen SEQ 1-308, wie z.B. in den Ansprüchen dargelegt, da die erfindungsgemäßen SEQ 1-308 wiederum Teilsequenzen, zumindest mit hoher Homologie, darstellen. Die spezifischen Markersequenzen SEQ 1-308 sind jedoch erfindungsgemäß bevorzugt.

Erfindungsgemäß umfassen die Markersequenzen auch solche Modifikationen der cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

In einer weiteren Ausführungsform der Erfindung sind ebenfalls Teilsequenzen oder Fragmente der erfindungsgemäßen Markersequenzen umfasst. Insbesondere solche Teilsequenzen, die eine Identität von 95%, 90 %, insbesondere 80% oder 70 % mit den erfindungsgemäßen Markersequenzen aufweisen.

Teilsequenzen sind ebenfalls solche Sequenzen, die 50 bis 100 Nukleotide, 70-120 Nukleotide einer Sequenz der SEQ 1-308 aufweisen, oder davon erhältliche Peptide.

"Teilsequenzen oder Fragmente" der erfindungsgemäßen Markersequenzen sind funktionell definiert und umfasst solche Sequenzen, die die gleiche erfindungsgemäße diagnostische Funktion aufweisen.

In einer weiteren Ausführungsform kann die jeweilige Markersequenz in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Ein weiterer Gegenstand der Erfindung betrifft eine Anordnung von Markersequenzen enthaltend mindestens eine Markersequenz einer cDNA ausgewählt aus der Gruppe SEQ 1 - 308 oder jeweils ein dafür kodierendes Protein. Vorzugsweise enthält die Anordnung mindestens 2 bis 5 oder 10, vorzugsweise 30 bis 50 Markersequenzen oder 50 bis 100 oder mehr Markersequenzen.

Im Rahmen dieser Erfindung bedeutet "Anordnung" synonym "Array" und sofern dieser "Array" zur Identifizierung von Substanzen an Markersequenzen verwendet wird, ist hierunter ein "Assay" oder eine diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Proteinbindern erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren. Ein Proteinbiochip im Sinne dieser Erfindung ist die systematische Anordnung von Proteinen auf einem festen Träger.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip bestehend aus einer Anordnung enthaltend erfindungsgemäße Markersequenzen.

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden.

Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einen festen Träger fixiert, gespottet oder immobilisiert.

Daher betrifft die Erfindung eine Anordnung, wobei die Markersequenzen als Clone vorliegen.

Zusätzlich können die Markersequenzen in der jeweiligen Form in Form eines Fusionsproteins vorliegen, welches beispielsweise mindestens ein Affinitätsepiptop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz für Multiple Sklerose, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Ferner betrifft die Erfindung ein Verfahren zum Identifizieren und Charakterisieren einer Substanz für Multiple Sklerose, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Die zu untersuchende Substanz kann ein beliebiges natives oder nicht-natives Biomolekül, ein synthetisches chemisches Molekül, eine Mischung oder eine Substanzbibliothek sein.

Nachdem die zu untersuchende Substanz eine Markersequenz kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Multiple Sklerose entwickelt und erhältlich durch den Einsatz des erfindungsgemäßen Assays oder Proteinbiochip.

Daher betrifft die Erfindung ebenfalls die Verwendung einer erfindungsgemäßen Anordnung oder einem Assay zum Screenen von Wirkstoffen für Multiple Sklerose.

Daher betrifft die Erfindung in einer weiteren Ausführungsform ebenfalls ein Target zur Behandlung und Therapie von Multiple Sklerose, jeweils ausgewählt aus der Gruppe SEQ 1 - 308 oder jeweils ein dafür kodierendes Protein.

In einer weiteren Ausführungsform betrifft die Erfindung ebenfalls die Verwendung der erfindungsgemäßen Markersequenzen, vorzugsweise in Form einer Anordnung, als Affinitätsmaterial zur Durchführung einer Apherese bzw. iwS. einer Blutwäsche, wobei Substanzen aus Körperflüssigkeiten eines Patienten mit Multiple Sklerose, wie Blut oder Plasma, an die erfindungsgemäßen Markersequenzen binden und folglich der Körperflüssigkeit selektiv entzogen werden können.

Beispiele und Figuren:
Zehn oder mehr Patientenproben wurden individuell gegen eine cDNA Expressionsbibliothek gescreent. Die Multiple Sklerose - spezifischen Expressionsklone wurden ermittelt durch einen Vergleich mit zehn oder mehr gesunden Proben. Die Identität der Markersequenzen wurde durch DNA-Sequenzierung ermittelt.

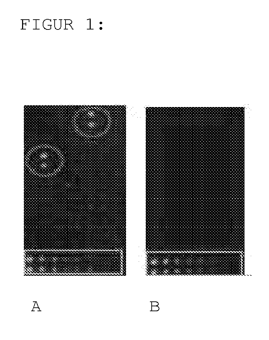

In Figur 1 wird das differentielle Screenen zwischen zwei Proteinbiochips aus jeweils einer cDNA-Expressionsbank eines Patienten und einem gesunden Probanden gezeigt. Die differentiellen Clone werden mittels Fluoresenzmarkierung nachgewiesen und bioinformatorisch ausgewertet.

Im Rahmen der Biomarkeridentifizierung werden verschiedene bioinformatische Analysen durchgeführt. Für jedes Serum werden mittels Microarray Reaktivitäten gegen ca. 2000 unterschiedliche Antigene gemessen. Diese Daten werden für ein Ranking der gespotteten Antigene bzgl. ihrer Differenzierungsfähigkeit zwischen gesunden und erkrankten Seren benutzt. Diese Auswertung wird mittels des nicht parametrischen Mann-Whitney Tests auf normalisierten Intensitätsdaten durchgeführt. Zur Normalisierung wird ein interner Standard benutzt, der auf jedem Chip mitgespottet wird. Da für jedes Antigen ein p-Wert berechnet wird, werden Methoden zur Korrektur des multiples Testens eingesetzt. Als sehr konservativer Ansatz wird eine Bonferroni Korrektur durchgeführt und zusätzlich wird die weniger restriktive False Discovery Rate (FDR) nach Benjamini & Hochberg berechnet.

Desweiteren werden die Daten zur Klassifikation der Seren benutzt. Hierbei kommen unterschiedliche multivariate Methoden zum Einsatz. Dies sind Methoden aus den statistischen Lernverfahren wie Support Vector Machines (SVM), Neuronale Netze oder Klassifikationsbäume, sowie eine Schwellenwertmethode, welche sowohl zur Klassifikation als auch zur visuellen Repräsentation der Daten geeignet ist.

Zur Vermeidung von Overfitting wird eine 10fache Cross-Validierung der Daten durchgeführt.

**Tabelle A: (gi Accession Nummer mit Geltung vom 1.10.2010)**

| SEQ 1b-308b | SEQ 1a-308a | |
|---|---|---|
| gi Acc Protein | gi Acc cDNA | NAME |
| gi\|157266266 | gi\|157266265 | WD repeat-containing protein 86 [Homo sapiens] |
| gi\|63252910 | gi\|63252909 | DDB1- and CUL4-associated factor 6 isoform b [Homo sapiens] |
| gi\|18699734 | gi\|20357519 | uridine-cytidine kinase 2 [Homo sapiens] |
| gi\|4505677 | gi\|260436978 | calcium/calmodulin-dependent 3',5'-cyclic nucleotide phosphodiesterase 1 B isoform 1 [Homo sapiens] |
| gi\|51477716 | gi\| 51477715 | alpha-mannosidase 2x [Homo sapiens] |
| gi\|24797097 | gi\|24797096 | pyrroline-5-carboxylate reductase 1, mitochondrial isoform 1 [Homo sapiens] |
| gi\|61676188 | gi\|195963314 | E3 ubiquitin-protein ligase HUWE1 [Homo sapiens] |
| gi\|30795119 | gi\|30795118 | F-box only protein 18 isoform 2 [Homo sapiens] |
| gi\|33469964 | gi\|33469963 | splicing factor 4 [Homo sapiens] |
| gi\|83035136 | gi\|217272875 | F-box only protein 31 [Homo sapiens] |
| gil6005747 | gi\|54792140 | E3 ubiquitin-protein ligase RING2 [Homo sapiens] |
| gil33636722 | gil261278365 | lipid phosphate phosphatase-related protein type 4 isoform 1 [Homo sapiens] |
| gi\|145199237 | gi\|145199236 | RNA exonuclease 1 homolog [Homo sapiens] |
| gi\|24308201 | gi\|41327713 | adipocyte plasma membrane-associated protein [Homo sapiens] |
| gi\|33356547 | gi\|33356546 | DNA replication licensing factor MCM2 [Homo sapiens] |
| gi\|7662074 | gi\|209413745 | zinc finger and BTB domain-containing protein 5 [Homo sapiens] |
| gi\|6005924 | gi\|115583673 | downregulated in renal cell carcinoma [Homo sapiens] |
| gi\|4505685 | gi\|291084749 | pyruvate dehydrogenase E1 component subunit alpha, somatic form, mitochondrial isoform 1 precursor [Homo sapiens] |
| gi\|20070228 | gi\|39725676 | nucleobindin-1 precursor [Homo sapiens] |
| gi\|21700763 | gi\|46361989 | hematological and neurological expressed 1-like protein (Homo sapiens] |
| gi\|19923927 | gi\|221139831 | general transcription factor 3C polypeptide 6 [Homo sapiens] |
| gi\| 26051235 | gi\|26051234 | nuclear pore complex protein Nup133 [Homo sapiens] |
| gi\|50593021 | gi\|50593020 | NFU1 iron-sulfur cluster scaffold homolog, mitochondrial isoform 2 [Homo sapiens] |
| gi\|38454194 | gi\|38454193 | gamma-tubulin complex component 4 [Homo sapiens] |
| gi\|5902122 | gi\|197381953 | spectrin beta chain, brain 2 [Homo sapiens] |
| gi\|62739181 | gi\|62739180 | rhotekin isoform c [Homo sapiens] |
| gi\|215599981 | gi\|215599980 | cyclin-D-binding Myb-like transcription factor 1 isoform b [Homo sapiens] |
| gi\|134288890 | gi\|148596939 | DIS3-like exonuclease 2 [Homo sapiens] |
| gi\|32698750 | gi\|32698749 | SR-related CTD-associated factor 1 [Homo sapiens] |
| gi\|4501887 | gi\|11038618 | actin, cytoplasmic 2 [Homo sapiens] |
| gi\|4758112 | gi\|93588182 | spliceosome RNA helicase BAT1 [Homo sapiens] |
| gi\|58331179 | gi\|58331178 | rho GTPase-activating protein 39 [Homo sapiens] |
| gi\|27477111 | gi\|119393888 | pre-mRNA-splicing factor SLU7 [Homo sapiens] |
| gi\|145309326 | gi\|145309325 | laminin subunit gamma-1 precursor [Homo sapiens] |
| gi\|4507145 | gi\|23111044 | sorting nexin-4 [Homo sapiens] |
| gi\|284055255 | gi\|284055254 | CM P-N-acetylneuraminate-beta-1,4-galactoside alpha-2,3-sialyltransferase isoform a |
| gi\|33356547 | gi\|33356546 | DNA replication licensing factor MCM2 [Homo sapiens] |
| gi\|13259508 | gi\|13259507 | dynactin 1 isoform 2 [Homo sapiens] |
| gi\|24797103 | gi\|24797102 | RAS guanyl releasing protein 2 [Homo sapiens] |
| gi\|20070228 | gi\|39725676 | nucleobindin 1 [Homo sapiens] |
| gi\|4502101 | gi\|4502100 | annexin I [Homo sapiens] |
| gi\|16975484 | gi\|92091602 | centaurin delta 2 isoform b [Homo sapiens] |
| gi\|21707902 | gil21707901 | CTTN protein [Homo sapiens] |
| gi\|29788785 | gi\|34222261 | tubulin, beta [Homo sapiens] |
| gi\|28827795 | gi\|40549398 | charged multivesicular body protein 4b [Homo sapiens] |
| gi\|149363636 | gi\|149363635 | plexin-B2 precursor [Homo sapiens] |
| gi\|66346681 | gi\|66346680 | plasminogen activator inhibitor 1 RNA-binding protein isoform 2 [Homo sapiens] |
| gi\|40548422 | gi\|40548421 | charged multivesicular body protein 4a [Homo sapiens] |
| gi\|3005715 | gi\|3005714 | protein 4.1-G [Homo sapiens] |
| gi\|22035672 | gi\|87196331 | thioredoxin reductase 2 precursor [Homo sapiens] |
| gi\|4506723 | gi\|70609888 | ribosomal protein S3a [Homo sapiens] |
| gi\|34485727 | gi\|153792638 | NCK-associated protein 1-like [Homo sapiens] |
| gi\|6912602 | gi\|38569401 | arfaptin-2 [Homo sapiens] |
| gi\|4506685 | gi\|14591910 | 40S ribosomal protein S13 [Homo sapiens] |
| gi\|18104948 | gi\|78190465 | 60S ribosomal protein L21 [Homo sapiens] |
| gi\|167466201 | gi\|167466200 | WAS protein family homolog 1 [Homo sapiens] |
| gi\|24234688 | gil156071496 | stress-70 protein, mitochondrial precursor [Homo sapiens] |
| gi\|307574659 | gi\|307574658 | hypothetical protein LOC116328 isoform 2 [Homo sapiens] |
| gi\|4506619 | gi\|78190466 | 60S ribosomal protein L24 [Homo sapiens] |
| gi\|94536842 | gi\|94536841 | ribose 5-phosphate isomerase A [Homo sapiens] |
| gi\|4505753 | gi\|31543395 | phosphoglycerate mutase 1 [Homo sapiens] |
| gi\|11545918 | gi\|210147465 | tubulointerstitial nephritis antigen-like 1 [Homo sapiens] |
| gi\|163644321 | gi\|163644320 | cytochrome b-c1 complex subunit Rieske, mitochondrial [Homo sapiens] |
| gi\|34526674 | gi\|34526673 | unnamed protein product [Homo sapiens] |
| gi\|4505573 | gi\|166064031 | rho guanine nucleotide exchange factor 7 isoform a [Homo sapiens] |
| gi\|5902122 | gi\|197381953 | spectrin beta chain, brain 2 [Homo sapiens] |
| gi\|23618848 | gi\|56676380 | protein SYS1 homolog isoform a [Homo sapiens] |
| gi\|83641870 | gi\|262331549 | nucleophosmin isoform 3 [Homo sapiens] |
| gi\|54112429 | gi\|54112428 | dedicator of cytokinesis protein 7 [Homo sapiens] |
| gi\|5453690 | gi\|5453689 | dnaJ homolog subfamily B member 1 [Homo sapiens] |
| gi\|10863945 | gi\|195963391 | X-ray repair cross-complementing protein 5 [Homo sapiens] |
| gi\|16753215 | gi\|94538348 | profilin-2 isoform a [Homo sapiens] |
| gi\|149363636 | gi\|149363635 | plexin-B2 precursor [Homo sapiens] |
| gi\|27501458 | gi\|157951660 | chromosome transmission fidelity protein 18 homolog [Homo sapiens] |
| gi\|205277463 | gi\|306518580 | transketolase [Homo sapiens] |
| gi\|21396500 | gi\|300116295 | HIRA interacting protein 3 [Homo sapiens] |
| gi\|71565154 | gi\|71565153 | alcohol dehydrogenase class-3 [Homo sapiens] |
| gi\|34147630 | gi\|169658370 | elongation factor Tu, mitochondrial precursor [Homo sapiens] |
| gi\|5729875 | gi\|216547928 | membrane-associated progesterone receptor component 1 [Homo sapiens] |
| gi\|50592996 | gi\|50592995 | tubulin beta-3 chain [Homo sapiens] |
| gi\|5802966 | gi\|58530846 | destrin isoform a [Homo sapiens] |
| gi\|19923315 | gi\|261862340 | serine hydroxymethyltransferase, mitochondrial isoform 1 precursor [Homo sapiens] |
| gi\|31982933 | gi\|33946335 | DNA-binding protein inhibitor ID-2 [Homo sapiens] |
| gi\|4506713 | gi\|294459919 | ubiquitin-40S ribosomal protein S27a precursor [Homo sapiens] |
| gi\|16753227 | gi\|67189547 | 60S ribosomal protein L6 [Homo sapiens] |
| gi\|50592996 | gi\|50592995 | tubulin beta-3 chain [Homo sapiens] |
| gi\|55743075 | gi\|96322659 | angio-associated migratory cell protein [Homo sapiens] |
| gi\|13569962 | gi\|116014337 | ras-related protein Rab-1 B [Homo sapiens] |
| gi\|78395056 | gi\|78395055 | C15orf23 protein [Homo sapiens] |
| gi\|45439359 | gi\|45439358 | triple functional domain protein [Homo sapiens] |
| gi\|34335253 | gi\|254911094 | disks large-associated protein 4 isoform a [Homo sapiens] |
| gi\|34098946 | gi\|109134359 | nuclease-sensitive element-binding protein 1 [Homo sapiens] |
| gi\|195972909 | gi\|195972908 | nasal embryonic luteinizing hormone-releasing hormone factor isoform a [Homo sapiens] |
| gi\|8394499 | gi\|283945567 | ubiquitin-associated protein 1 isoform 1 [Homo sapiens] |
| gi\|24234688 | gi\|296080701 | stress-70 protein, mitochondrial precursor [Homo sapiens] |
| gi\|4758138 | gi\|221139768 | probable ATP-dependent RNA helicase DDX5 [Homo sapiens] |
| gi\|34335251 | gi\|109891935 | disks large-associated protein 4 isoform b [Homo sapiens] |
| gi\|11342680 | gi\|197245402 | beta-centractin [Homo sapiens] |
| gi\|5453832 | gi\|169234641 | hypoxia up-regulated protein 1 precursor [Homosapiens] |
| gi\|34098946 | gi\|109134359 | nuclease-sensitive element-binding protein 1 [Homo sapiens] |
| gi\|154090959 | gi\|154090958 | WASH complex subunit FAM21 B [Homo sapiens] |
| gi\|4506913 | gi\|209693454 | beta-sarcoglycan [Homo sapiens] |
| gi\|247425318 | gi\|247425317 | immunoglobulin heavy chain variable region [Homo sapiens] |
| gi\|503170 1 | gi\|95104789 | follistatin-like 3 (secreted glycoprotein) |
| gi\|13375616 | gi\|34304362 | fatty acid desaturase 3 [Homo sapiens] |
| gi\|13376888 | gi\|34147389 | transmembrane protein 121 [Homo sapiens] |
| gi\|17986283 | gi\|117986282 | tubulin alpha-1A chain [Homo sapiens] |
| gi\|5031669 | gi\|39725675 | cyclin-dependent kinase 2-associated protein 2 [Homo sapiens] |
| gi\|14249132 | gi\|270309185 | protein BEX2 isoform 3 [Homo sapiens] |
| gi\|22027541 | gi\|22027540 | programmed cell death protein 7 [Homo sapiens] |
| gi\|4507761 | gi\|7539056 | ubiquitin-60S ribosomal protein L40 precursor [Homo sapiens] |
| gi\|9353009 | gi\|19353008 | Similar to Elongation factor 2b [Homo sapiens] |
| gi\|110815842 | gi\|34147354 | dysbindin domain-containing protein 1 isoform 2 [Homo sapiens] |
| gi\|171846268 | gi\|291084495 | elongation factor Ts, mitochondrial isoform 2 precursor [Homo sapiens] |
| gi\|4557325 | gi\|48762938 | apolipoprotein E precursor [Homo sapiens] |
| gi\|41406055 | gi\|228008404 | amyloid beta A4 protein isoform b precursor [Homo sapiens]. |
| gi\|23510421 | gi\|23510420 | tumor necrosis factor receptor superfamily; member 6 isoform 2 precursor [Homo sapiens] |
| gi\|15718706 | gi\|122056469 | caspase 8 isoform B precursor [Homo sapiens] |
| gi\|119575060 | | Homo sapiens CD24 molecule (CD24) |
| gi\|17978489 | gi\|68508947 | Homo sapiens CD97 molecule (CD97); transcript variant 1 |
| gi\|52630326 | gi\|158420733 | chromodomain helicase DNA binding protein 3 [Homo sapiens] |
| gi\|4503481 | gi\|83656774 | eukaryotic translation elongation factor 1 gamma [Homo sapiens] |
| gi\|116063573 | gi\|160420313 | filamin A; alpha isoform 1 [Homo sapiens]. |
| gi\|4503979 | gi\|96115280 | glial fibrillary acidic protein isoform 1 [Homo sapiens] |
| gi\|150418002 | gi\|150418001 | HLA class II histocompatibility antigen, DQ beta 1 chain precursor [Homo sapiens] |
| gi\|55925614 | gi\|55925613 | interferon alpha-inducible protein 27, mitochondrial isoform 2 [Homo sapiens] |
| gi\|10835145 | gi\|27894305 | interleukin 1; beta proprotein [Homo sapiens] |
| gi\|28610151 | gi\|28610150 | interleukin-7 receptor subunit alpha precursor [Homo sapiens] |
| gi\|119703755 | gi\|119703754 | laminin; beta 2 precursor [Homo sapiens]. |
| gi\|5031877 | gi\|27436949 | Lamin B1 [Homo sapiens] |
| gi\|82534351 | gi\|189409155 | microtubule-associated protein tau isoform 1 |
| gi\|4505241 | gi\|98991774 | protein Mpv17 [Homo sapiens] |
| gi\|222136617 | gi\|222136616 | interferon-induced GTP-binding protein Mx1 [Homo sapiens] |
| gi\|105990539 | gi\|197927150 | neurofilament; light polypeptide 68kDa [Homo sapiens] |
| gi\|27886561 | gi\|27886560 | nuclear factor of activated T-cells, cytoplasmic 3 isoform 1 [Homo sapiens] |
| gi\|205360954 | gi\|205360953 | polycystin-1 isoform 1 precursor [Homo sapiens] |
| gi\|32171249 | gi\|38505192 | prostaglandin-H2 D-isomerase [Homo sapiens] |
| gi\|18641347 | gi\|115385975 | Homo sapiens protein tyrosine phosphatase; receptor type; C (PTPRC); transcript variant 1 |
| gi\|4506367 | gi\|209915550 | ras-related protein Rab-3A [Homo sapiens] |
| gi\|4506701 | gi\|71772514 | 40S ribosomal protein S23 [Homo sapiens] |
| gi\|4506841 | gi\|56119169 | C-C motif chemokine 2 precursor [Homo sapiens] |
| gi\|19557702 | gi\|157266286 | surfeit 6 [Homo sapiens] |
| gi\|21359969 | gi\|141801742 | DNA-directed RNA polymerase III subunit RPC3 [Homo sapiens] |
| gi\|5803227 | gi\|21464103 | 14-3-3 protein theta [Homo sapiens] |
| gi\|148747351 | gi\|296841089 | protein kinase C and casein kinase substrate in neurons 2 [Homo sapiens] |
| gi\|11321634 | gi\|125987597 | CD2-associated protein [Homo sapiens] |
| gi\|223468620 | gi\|223468619 | E3 ubiquitin-protein ligase makorin-1 isoform 1 [Homo sapiens] |
| gi\|24308113 | gi\|291190751 | KIF1 binding protein [Homo sapiens]. |
| gi\|82617630 | gi\|82617629 | Cytoplasmic FMR1 interacting protein 2 [Homo sapiens] |
| gi\|48949851 | gi\|48949850 | HERV-W_7q21.2 provirus ancestral Env polyprotein precursor [Homo sapiens] |
| gi\|7706702 | gi\|28144902 | interleukin-23 subunit alpha precursor [Homo sapiens] |
| gi\|115299754 | gi\|115299753 | dysbindin domain-containing protein 2 isoform b [Homo sapiens] |
| gi\|70887780 | gi\|70887779 | Homo sapiens sperm associated antigen 16 |
| gi\|119120897 | gi\|119120896 | partitioning defective 3 homolog B isoform b [Homo sapiens] |
| gi\|209969690 | gi\|209969689 | hypothetical protein LOC119032 [Homo sapiens] |
| gi\|187960086 | gi\|1187960086 | cytochrome P450 4V2 [Homo sapiens] |
| gi\|6978649 | gi\|242246959 | choline/ethanolamine kinase [Homo sapiens] |
| gi\|4506649 | gil76496470 | 60S ribosomal protein L3 isoform a [Homo sapiens] |
| gi\|27545326 | gi\|55956799 | SWI/SNF related, matrix associated, actin dependentregulator of chromatin, subfamily b, member 1 [Homo sapiens] |
| gi\|115527080 | gi\|115527079 | metastasis-associated protein MTA1 [Homo sapiens] |
| gi\|56788399 | gi\|56788398 | GI:56788398 |
| gi\|239787092 | gi\|239787091 | TNFAIP3-interacting protein 2 isoform 1 [Homo sapiens] |
| gi\|54112429 | gi\|54112428 | dedicator of cytokinesis protein 7 [Homo sapiens] |
| gi\|4504603 | gi\|50593016 | Interferon beta1 |
| gi\|4504605 | gi\|4504604 | Interferon omega |
| gi\|10834984 | gi\|224831235 | Interleukin IL-6 |
| gi\|10835141 | gi\|24430216 | Interleukin IL-10 |
| gi\|24430219 | gi\|24430218 | interleukin-12 subunit alpha precursor [Homo sapiens] |
| gi\|24497438 | gi\|24497437 | interleukin-12 subunit beta precursor [Homo sapiens] |
| gi\|25306235 | gi\|219842281 | brain-derived neurotrophic factor isoform b preproprotein [Homo sapiens] |
| gi\|4758020 | gi\|209574322 | ciliary neurotrophic factor [Homo sapiens] |
| gi\|10834978 | gi\|28610153 | interleukin-8 precursor (Homo sapiens] |
| gi\|89903008 | gi\|237858673 | neurofascin isoform 4 precursor [Homo sapiens] |
| gi\|17158044 | gi\|17158043 | ribosomal protein S6 [Homo sapiens] |
| gi\|51476647 | gi\|51476646 | Ankyrin repeat and SAM domain-containing protein 6 |
| gi\|72534660 | gi\|197209865 | splicing factor, arginine/serine-rich 7 [Homo sapiens] |
| gi\|13128968 | gi\|13128967 | dual specificity phosphatase 26 [Homo sapiens] |
| gi\|22538467 | gi\|22538466 | proteasome (prosome, macropain) subunit, beta type, 4 [Homo sapiens] |
| gi\|15055539 | gi\|70609878 | ribosomal protein S2 [Homo sapiens] |
| gi\|13129006 | gi\|13129005 | DEAD (Asp-Glu-Ala-Asp) box polypeptide 50 (Homo sapiens] |
| gi\|4506663 | gi\|2377361 | ribosomal protein L8 [Homo sapiens] |
| gi\|19923193 | gi\|21237722 | suppression of tumorigenicity 13 (colon carcinoma) (Hsp70 interacting protein) (ST13) |
| gi\|4506649 | gi\|76496470 | ribosomal protein L3 isoform a [Homo sapiens] |
| gi\|4506743 | gi\|4506742 | ribosomal protein S8 [Homo sapiens] |
| gi\|5031877 | gi\|27436949 | lamin B1 [Homo sapiens] |
| gi\|94536842 | gi\|94536841 | ribose 5-phosphate isomerase A [Homo sapiens] |
| gi\|17157993 | gi\|141803509 | olfactomedin 2 [Homo sapiens] |
| gi\|8922911 | gi\|8922910 | radical S-adenosyl methionine domain containing 1 [Homo sapiens] |
| gi\|4826724 | gi\|17105402 | zygin 1 isoform 1 [Homo sapiens] |
| gi\|6806913 | gi\|187960101 | centaurin, alpha 1 [Homo sapiens] |
| gi\|5454058 | gi\|5454057 | ST3 beta-galactoside alpha-2,3-sialyltransferase 4 [Homo sapiens] |
| gi\|54607091 | gi\|54607090 | SUMO1/sentrin/SMT3 specific protease 2 [Homo sapiens] |
| gi\|166063995 | gi\|166063994 | general transcription factor IIIA [Homo sapiens] |
| gi\|72534684 | gi\|166197669 | phospholipase D family, member 3 [Homo sapiens] |
| gi\|14591909 | gi\|1772259 | ribosomal protein L5 [Homo sapiens] |
| gi\|11415026 | gi\|15431299 | ribosomal protein L18a [Homo sapiens] |
| gi\|13129004 | gi\|30089943 | GIY-YIG domain containing 2 isoform 1 [Homo sapiens] |
| gi\|62414289 | gi\|240849334 | vimentin [Homo sapiens] |
| gi\|110347461 | gi\|110347460 | MYC-associated zinc finger protein isoform 1 [Homo sapiens] |
| gi\|4758648 | gi\|187761329 | kinesin family member 5B [Homo sapiens] |
| gi\|4502337 | gi\|38372939 | alpha-2-glycoprotein 1, zinc-binding [Homo sapiens] |
| gi\|6912642 | gi\|142383813 | sex comb on midleg 1 isoform 2 [Homo sapiens] |
| gi\|4503065 | gi\|62241006 | crystallin, mu isoform 1 [Homo sapiens] |
| gi\|4505409 | gi\|66392201 | nucleoside diphosphate kinase B [Homo sapiens] |
| gi\|4507791 | gi\|150417997 | ubiquitin-conjugating enzyme E2M (UBC12 homolog, yeast) |
| gi\|7657015 | gi\|187936926 | hypothetical protein LOC51493 [Homo sapiens] |
| gi\|16554609 | gi\|16554608 | 28S ribosomal protein S11, mitochondrial isoform a [Homo sapiens] |
| gi\|2972561 | gi\|30065641 | serine/threonine-protein phosphatase 2A activator isoform b [Homo sapiens] |
| gi\|5902082 | gi\|151101481 | ST3 beta-galactoside alpha-2,3-sialyltransferase 2 [Homo sapiens] |
| gi\|29893564 | gi\|34222264 | microspherule protein 1 isoform 1 [Homo sapiens] |
| gi\|19923796 | gi\|142359942 | 60S ribosomal export protein NMD3 [Homo sapiens] |
| gi\|4506661 | gi\|18390348 | ribosomal protein L7a [Homo sapiens] |
| gi\|16579885 | gi\|16579884 | 60S ribosomal protein L4 [Homo sapiens] |
| gi\|40548389 | gi\|66346686 | dickkopf homolog 3 precursor [Homo sapiens] |
| gi\|5453880 | gi\|221219065 | acidic (leucine-rich) nuclear phosphoprotein 32 family, member A [Homo sapiens] |
| gi\|22035558 | gi\|148833509 | transcription factor IIIB 90 kDa subunit isoform 3 [Homo sapiens] |
| gi\|4506617 | gi\|78000184 | ribosomal protein L17 [Homo sapiens] |
| gi\|27545323 | gil168229166 | chondroitin polymerizing factor [Homo sapiens] |
| gi\|162750347 | gi\|62750346 | histone deacetylase 5 isoform 1 [Homo sapiens] |
| gi\|100913206 | gi\|100913205 | ATP-dependent RNA helicase A [Homo sapiens] |
| gi\|106879210 | gi\|106879209 | SH2 domain-containing adapter protein B [Homo sapiens] |
| gi\|18152783 | gi\|18490985 | 60S ribosomal protein L10-like [Homo sapiens] |
| gi\|15431301 | gi\|72187675 | ribosomal protein L7 [Homo sapiens] |
| gi\|13375618 | gi\|114155130 | 24-dehydrocholesterol reductase precursor [Homo sapiens] |
| gi\|14043070 | gi\|83641894 | heterogeneous nuclear ribonucleoprotein A1 [Homo sapiens] |
| gi\|22202633 | gi\|88999578 | prefoldin subunit 5 isoform alpha [Homo sapiens] |
| gi\|22907052 | gi\|300360513 | actin-related protein 2/3 complex subunit 1 A isoform 1 [Homo sapiens] |
| gi\|23308577 | gi\|217272837 | D-3-phosphoglycerate dehydrogenase [Homo sapiens] |
| gi\|34098946 | gi\|109134359 | nuclease-sensitive element-binding protein 1 [Homo sapiens] |
| gi\|4502015 | gi\|109637793 | A-kinase anchor protein 1 precursor [Homo sapiens] |
| gi\|4502027 | gi\|215982788 | albumin preproprotein [Homo sapiens] |
| gi\|4506631 | gi\|15812218 | 60S ribosomal protein L30 [Homo sapiens] |
| gi\|4506667 | gi\|49087144 | 60S acidic ribosomal protein PO [Homo sapiens] |
| gi\|4508007 | gi\|197382778 | zinc finger protein 174 isoform a [Homo sapiens] |
| gi\|5031851 | gi\|44889961 | stathmin isoform a [Homo sapiens] |
| gi\|52630322 | gi\|158420732 | chromodomain-helicase-DNA-binding protein 3 isoform 2 [Homo sapiens] |
| gi\|87080813 | gi\|87080812 | LON peptidase N-terminal domain and ring finger 1 [Homo sapiens] |
| gi\|9945439 | gi\|90193629 | septin-5 [Homo sapiens] |
| gi\|5803013 | gi\|77628146 | endoplasmic reticulum protein 29 isoform 1 precursor [Homo sapiens] |
| gi\|168229248 | gi\|168229247 | asparagine synthetase [Homo sapiens] |
| gi\|90652861 | gi\|90652860 | protein tyrosine phosphatase, non-receptor type 5 (striatum-enriched) isoform b [Homo sapiens] |
| gi\|58761502 | gi\|58761501 | GTP-binding protein PTD004 isoform 2 [Homo sapiens] |
| gi\|94538370 | gi\|94538369 | DnaJ (Hsp40) homolog, subfamily C, member 2 isoform 1 [Homo sapiens] |
| gi\|10047104 | gi\|110227859 | synovial sarcoma translocation gene on chromosome 18-like 2 [Homo sapiens] |
| gi\|9951915 | gi\|239937553 | S-adenosylhomocysteine hydrolase [Homo sapiens] |
| gi\|4507729 | gi\|68299771 | tubulin, beta 2 [Homo sapiens] |
| gi\|5031875 | gi\|153281091 | lamin A/C isoform 2 [Homo sapiens] |
| gi\|41393561 | gi\|41393560 | leucine aminopeptidase 3 [Homo sapiens] |
| gi\|157885806 | gi\|157885805 | chromosome 12 open reading frame 51 [Homo sapiens] |
| gi\|119624431 | 0 | hCG2041192 [Homo sapiens] |
| gi\|112382250 | gi\|112382249 | spectrin, beta, non-erythrocytic 1 isoform 1 [Homo sapiens] |
| gi\|93141029 | gi\|93141028 | paralemmin isoform 2 [Homo sapiens] |
| gi\|4501867 | gi\|46411160 | aconitase 2 precursor [Homo sapiens] |
| gi\|88758580 | gi\|221554513 | cyclin L2 isoform A [Homo sapiens] |
| gi\|5901922 | gi\|39995072 | cell division cycle 37 protein [Homo sapiens] |
| gi\|21624607 | gi\|23510452 | coactosin-like 1 [Homo sapiens] |
| gi\|24234747 | gi\|24234746 | interleukin enhancer binding factor 2 [Homo sapiens] |
| gi\|160420328 | gi\|160420327 | iron-sulfur cluster assembly 2 [Homo sapiens] |
| gi\|6005743 | gi\|62241020 | DEAD (Asp-Glu-Ala-As) box polypeptide 19 isoform 1 [Homo sapiens] |
| gi\|15010818 | gi\|15010817 | JKTBP1delta6 [Homo sapiens] |
| gi\|4502847 | gi\|186972139 | cold inducible RNA binding protein [Homo sapiens] |
| gi\|71164894 | gi\|71164893 | trinucleotide repeat containing 4, isoform CRA_a [Homo sapiens] |
| gi\|4758206 | gi\|187608704 | dual specificity phosphatase 2 [Homo sapiens] |
| gi\|5730009 | gi\|115387097 | ret finger protein [Homo sapiens] |
| gi\|7657689 | gi\|21327683 | YME1-like 1 isoform 3 [Homo sapiens] |
| gi\|28872792 | gi\|28872791 | CDK5 regulatory subunit associated protein 3 [Homo sapiens] |
| gi\|15147333 | gi\|189458899 | tripartite motif-containing 37 protein [Homo sapiens] |
| gi\|29826319 | gi\|29826318 | adducin 1 (alpha) isoform a [Homo sapiens] |
| gi\|190194416 | gi\|190194415 | F-box and leucine-rich repeat protein 15 [Homo sapiens] |
| gi\|4758272 | gi\|46389548 | endosulfine alpha isoform 3 [Homo sapiens] |
| gi\|166795250 | gi\|166795249 | kinesin family member 2C [Homo sapiens] |
| gi\|151301215 | gi\|151301214 | widely-interspaced zinc finger motifs [Homo sapiens] |
| gi\|46048234 | gi\|194294559 | nucleolar protein 8 [Homo sapiens] |
| gi\|219842250 | gi\|219842249 | periphilin 1 isoform 8 [Homo sapiens] |
| gi\|48762942 | gi\|48762941 | huntingtin interacting protein-1-related [Homo sapiens] |
| gi\|4506003 | gi\|45827796 | protein phosphatase 1, catalytic subunit, alpha isoform 1 [Homo sapiens] |
| gi\|67782338 | gi\|67782337 | amyloid precursor-like protein 1 isoform 1 precursor [Homo sapiens] |
| gi\|17402896 | gi\|17402895 | RAD51 homolog C isoform 1 [Homo sapiens] |
| gi\|5453629 | gi\|34335254 | dynactin 2 [Homo sapiens] |
| gi\|11968182 | gi\|14165467 | ribosomal protein S18 [Homo sapiens] |
| gi\|122937289 | gi\|122937288 | kinesin family member 18B [Homo sapiens] |
| gi\|155029542 | gi\|1155029541 | BEN domain containing 7 isoform 1 [Homo sapiens] |
| gi\|11321585 | gi\|20357526 | guanine nucleotide-binding protein, beta-1 subunit [Homo sapiens] |
| gi\|12597635 | gi\|12597634 | B-cell CLL/lymphoma 11 B isoform 2 [Homo sapiens] |
| gi\|4502751 | gi\|17981697 | cyclin-dependent kinase inhibitor 2C (p18, inhibits CDK4), isoform CRA_b [Homo sapiens] |
| gi\|7662046 | gi\|7662045 | myeloid/lymphoid or mixed-lineage leukemia 4 [Homo sapiens] |
| gi\|37537687 | gi\|37537686 | zinc finger protein 444 [Homo sapiens] |
| gi\|133922582 | gi\|133922581 | zinc finger protein 358 [Homo sapiens] |
| gi\|189083826 | gi\|189083825 | inhibitor of growth family, member 4 isoform 3 [Homo sapiens] |
| gi\|110224479 | gi\|110224478 | prosaposin isoform c preproprotein [Homo sapiens] |
| gi\|21264343 | gil21264342 | scaffold attachment factor B [Homo sapiens] |
| gi\|14670375 | gi\|14670374 | SCG10-like-protein [Homo sapiens] |
| gi\|12545395 | gi\|94721348 | islet cell autoantigen 1 [Homo sapiens] |
| gi\|91199552 | gi\|91199551 | ADP-ribosylation factor-like protein 16 [Homo sapiens] |
| gi\|21614499 | gi\|161702984 | ezrin [Homo sapiens] |
| gi\|5453690 | gi\|5453689 | DnaJ (Hsp40) homolog, subfamily B, member 1 [Homo sapiens] |
| gi\|20070228 | gi\|39725676 | nucleobindin 1 [Homo sapiens] |
| gi\|12667788 | gi\|225703132 | myosin, heavy polypeptide 9, non-muscle [Homo sapiens] |
| gi\|5902158 | gi\|215422343 | ring finger protein 113A [Homo sapiens] |
| gi\|162241011 | gi\|62241010 | v-akt murine thymoma viral oncogene homolog 1 [Homo sapiens] |
| gi\|27734911 | gi\|27734910 | DAZ interacting protein 1-like [Homo sapiens] |
| gi\|7669492 | gi\|83641890 | glyceraldehyde-3-phosphate dehydrogenase [Homo sapiens] |
| gi\|7657514 | gi\|21314660 | GTP-binding protein RHO6 [Homo sapiens] |
| gi\|38257139 | gi\|38257138 | protein kinase, cAMP-dependent, regulatory, type 1, beta [Homo sapiens] |
| gi\|23503295 | gi\|26787971 | casein kinase 2, beta polypeptide [Homo sapiens] |
| gi\|20128774 | gi\|47519746 | mitogen-activated protein kinase 11 [Homo sapiens] |
| gi\|4759274 | gi\|215422360 | thioredoxin-like 1 [Homo sapiens] |
| gi\|32189394 | gi\|50345985 | ATP synthase, H+ transporting, mitochondrial F1 complex, beta subunit precursor [Homo sapiens] |

## Patentansprüche

1. Verwendung der Markersequenzen zur Diagnose von Multiple Sklerose, wobei mindestens eine Markersequenz einer cDNA ausgewählt aus der Gruppe SEQ 1 - 308 und / oder SEQ 1a-308a oder jeweils ein dafür kodierendes Protein oder jeweils einer Teilsequenz oder Fragment davon an oder von einem zu untersuchenden Patienten bestimmt wird.

2. Verwendung der Markersequenzen zur Diagnose von Multiple Sklerose nach Anspruch 1, **dadurch gekennzeichnet**, mindestens 2 bis 5 oder 10, vorzugsweise 30 bis 50 Markersequenzen oder 50 bis 100 oder mehr Markersequenzen an oder von einem zu untersuchenden Patienten bestimmt wird.

3. Verwendung der Markersequenzen zur Diagnose von Multiple Sklerose nach Anspruch 1 oder 2, **dadurch gekennzeichnet, dass** die Bestimmung mittels in-vitro Diagnose erfolgt.

4. Verwendung einer Markersequenz einer cDNA jeweils ausgewählt aus der Gruppe SEQ 1 - 308 und / oder SEQ 1a-308a oder jeweils ein dafür kodierendes Protein oder jeweils einer Teilsequenz oder Fragment davon als Diagnostikum.

5. Verwendung der Markersequenzen zur Diagnose von Multiple Sklerose nach einem der vorhergehenden Ansprüche, **dadurch gekennzeichnet, dass** die Markersequenzen auf einem festen Träger aufgebracht werden, insbesondere einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix.

6. Verfahren zur Diagnose von Multiple Sklerose, wobei
a.) mindestens eine Markersequenz einer cDNA ausgewählt aus der Gruppe SEQ 1 - 308 und / oder SEQ 1a-308a oder jeweils ein dafür kodierendes Protein oder jeweils einer Teilsequenz oder Fragment davon auf einem festen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

7. Verfahren zum Stratifizieren, insbesondere zur Risikostratifizierung, oder zur Therapiesteuerung eines Patienten mit Multiple Sklerose, wobei mindestens eine Markersequenz einer cDNA ausgewählt aus der Gruppe SEQ 1 - 308 und / oder SEQ 1a-308a oder jeweils ein dafür kodierendes Protein oder jeweils einer Teilsequenz oder Fragment davon an einem zu untersuchenden Patienten bestimmt wird.

8. Verfahren nach Anspruch 7, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

9. Anordnung von Markersequenzen enthaltend mindestens eine Markersequenz einer cDNA ausgewählt aus der Gruppe SEQ 1 - 308 und / oder SEQ 1a-308a oder jeweils ein dafür kodierendes Protein.

10. Anordnung nach Anspruch 9, **dadurch gekennzeichnet, dass** mindestens 2 bis 5 oder 10, vorzugsweise 30 bis 50 Markersequenzen oder 50 bis 100 oder mehr Markersequenzen enthalten sind.

11. Anordnung nach Anspruch 9, **dadurch gekennzeichnet, dass** die Markersequenzen als Clone vorliegen.

12. Assay, Proteinbiochip bestehend aus einer Anordnung nach Anspruch 9, **dadurch gekennzeichnet, dass** die Markersequenzen auf einem festen Träger aufgebracht sind.

13. Verwendung einer Anordnung nach einem der Ansprüche 9 bis 11 oder einem Assay nach Anspruch 12 zum Identifizieren und Charakterisieren einer Substanz für Multiple Sklerose enthaltend Mittel zum Nachweis eines Bindungserfolges, **dadurch gekennzeichnet, dass** eine Anordnung oder Assay mit a.) mindestens einer zu untersuchenden Substanz in Kontakt gebracht wird und b.) ein Bindungserfolg nachgewiesen wird.

14. Verwendung einer Anordnung nach einem der Ansprüche 9 bis 11 oder einem Assay nach Anspruch 12 zum Screenen von Wirkstoffen für Multiple Sklerose.

15. Diagnostika zur Diagnose von Multiple Sklerose, jeweils ausgewählt aus der Gruppe SEQ 1 - 308 und / oder SEQ 1a-308a oder jeweils ein dafür kodierendes Protein oder jeweils einer Teilsequenz oder Fragment davon.

16. Target zur Behandlung und Therapie von Multiple Sklerose, jeweils ausgewählt aus der Gruppe SEQ 1 - 308 und / oder SEQ 1a-308a oder jeweils ein dafür kodierendes Protein oder jeweils einer Teilsequenz oder Fragment davon.

17. Verwendung einer Markersequenz einer cDNA ausgewählt aus der Gruppe SEQ 1 - 308 und / oder SEQ 1a-308a oder jeweils ein dafür kodierendes Protein oder jeweils einer Teilsequenz oder Fragment davon als Affinitätsmaterial zur Durchführung einer Apherese oder Blutwäsche für Patienten mit Multiple Sklerose.
